# EUROPÄISCHE PATENTSCHRIFT

(11) **EP 3 897 781 B1**
(45) Veröffentlichungstag und Bekanntmachung des Hinweises auf die Patenterteilung: **07.12.2022**
(21) Anmeldenummer: 19801703.0
(22) Anmeldetag: 28.10.2019
(51) Int. Cl.: A61M 5/24, A61M 5/34, A61M 5/32

(54) **VORRICHTUNG FÜR DAS NADELROHR EINER SPRITZE**
DEVICE FOR THE NEEDLE PIPE OF A SYRINGE
DISPOSITIF POUR LE TUYAU D'AIGUILLE D'UNE SERINGUE

(30) Priorität: 18.12.2018 DE 202018107232 U
(43) Veröffentlichungstag der Anmeldung: 27.10.2021
(73) Patentinhaber: Fischer, Stephan, 32120 Hiddenhausen (DE); Wilke, Tobias, 49477 Ibbenbüren (DE); Mohr, Bernd, 25355 Barmstedt (DE)
(72) Erfinder: Fischer, Stephan, 32120 Hiddenhausen (DE); Wilke, Tobias, 49477 Ibbenbüren (DE); Mohr, Bernd, 25355 Barmstedt (DE)
(74) Vertreter: Pellengahr, Maximilian Rudolf
(86) Internationale Anmeldenummer: PCT/EP2019/079383
(87) Internationale Veröffentlichungsnummer: WO 2020/126176

(56) Entgegenhaltungen:
- GB-A- 2 532 979
- US-A- 4 976 701
- US-A1- 2014 135 713
- US-A1- 2018 344 934

## Beschreibung

### Einleitung

Die Erfindung betrifft eine Vorrichtung für das Nadelrohr einer Spritze, umfassend ein an einem Trägerelement für das Nadelrohr schwenkbar ausgebildetes Gehäuse, welches in der Schwenkebene des Gehäuses eine offene Gehäuseseite umfasst, so dass das Nadelrohr nach Injektionsgebrauch zur Aufnahme in das Gehäuse eingeschwenkt ist.

### Stand der Technik

Es ist bekannt, dass die Nadel einer Injektionsspritze ein mit einem hohlen Trägerelement verbundenes, mit einer Spritze versehenes Metallrohr umfasst, das mit dem zylindrischen Körper einer Spritze verbunden ist. Die vorliegende Erfindung geht aus von einem Metallrohrschutz gegen Nadelstiche, insbesondere nach dem eine Injektion in den Körper eines Patienten durchgeführt wurde. Um Infektionen an Körpern zu vermeiden, die mit der Nadel nach der Behandlung in Berührung kommen könnten. Wenn also eine zweite Person mit Nadeln gestochen würde, nachdem sie bei einer ersten Person verwandt wurde, kann es zur Übertragung von Krankheiten kommen. Daher ist mit dem Gebrauch dieser Nadel eine beträchtliche Gefahr, insbesondere für diejenigen verbunden, die normalerweise mit ihnen arbeiten, insbesondere für Krankenhaus- und Arztpersonal. Die Entsorgung von benutzten Nadeln als Abfall birgt auch eine Gefahr für diejenigen, die mit den Abfallbehältern hantieren oder sich diesen Behältern nähern. Daher verfügt die Vorrichtung für das Nadelrohr ein an einem Trägerelement schwenkbar ausgebildetes Gehäuse, so dass nach Gebrauch des Nadelrohrs diese wieder zum Schutz in das Gehäuse verschwenkt werden kann.

Die Erfindung geht hierbei insbesondere von dem Trägerelement aus, wobei an das Trägerelement bzw. auf das Nadelrohr die Auslassdüse beispielsweise einer Spritze gesteckt wird, um so den Infusionsvorgang über die Betätigung des Kolbens der Spritze vorzunehmen. Jedoch wird es zunehmend gewünscht, dass beispielsweise auch sogenannte Ampullen oder Behältnisse, in denen das Injektionsmittel vorgehalten wird, mit der Vorrichtung verbunden wird, um auf diese Weise eine Injektion vorzuhalten.

In dem Dokument GB 2532979 A ist eine Vorrichtung für das Nadelrohr einer Spritze beschrieben, umfassend ein an einem Trägerelement für das Nadelrohr schwenkbar ausgebildetes Gehäuse, welches in der Schwenkebene des Gehäuses eine offene Gehäuseseite aufweist, sodass das Nadelrohr nach Injektionsgebrauch zur Aufnahme in das Gehäuse einschwenkbar ist, wobei das Trägerelement ein Gehäuse umfasst.

### Aufgabe

Der Erfindung stellt sich somit das Problem, eine Vorrichtung für das Nadelrohr einer Spritze, welches ein Trägerelement mit einem schwenkbar ausgebildeten Gehäuse umfasst derart weiterzubilden, welches geeignet ist, Ampullenbehältnisse oder auch andere Behältnisse beinhaltende Substanzen derart vorzuhalten, dass diese sicher an das Trägerelement andockbar sind.

### Lösung

Erfindungsgemäß wird dieses Problem mit dem Merkmal des Hauptanspruchs gelöst, vorteilhafte Ausgestaltungen der Erfindung ergeben sich aus den Unteransprüchen.

Die mit der Erfindung erreichten Vorteile bestehen darin, dass die erfindungsgemäße Vorrichtung für das Nadelrohr einer Spritze ein Trägerelement aufweist, in das der Stutzen einer Ampulle so eingeführt werden kann, dass einerseits ein sicheres Andocken sowie eine sichere Entnahme der Flüssigkeit durch das Nadelrohr vorgenommen werden kann.

Erfindungsgemäß wird hierzu vorgeschlagen, dass die Anordnung des freien Endes des Nadelrohres im Trägerelement ein kontrolliertes Durchstoßen der Stutzenwand eines in das Trägerelement einsetzbaren Behältnisses ermöglicht. Wird also das in Form einer Ampulle vorgehaltene Behältnis in das Trägerelement eingeführt, so ergibt sich ein selbsttätiger Anstich des Behältnisses zur Entnahme der Injektionsflüssigkeit.

In Weiterbildung erfolgt hierbei der kontrollierte Durchstoßvorgang des freien Endes des Nadelrohrs durch eine Schwenk- oder Drehbewegung des eingesetzten Behältnisses gegenüber dem Trägerelement. Diese Ausbildung ermöglicht es nun, dass, wenn das Behältnis mit seinem Stutzen sicher im Trägerelement eingeführt ist, durch eine Betätigung in Form einer Schwenkbewegung oder einer Drehbewegung ein sicherer Anstich des Behältnisses vorgenommen wird, ohne dass die Injektionsflüssigkeit durch eine Leckage austreten kann.

Das Trägerelement umfasst hierbei ein Gehäuse, in dem ein drehbar gelagertes axial verschiebbares Innenteil angeordnet ist. In Weiterbildung steht hierbei das Innenteil über eine Kulissenführung mit dem Gehäuse in Wirkverbindung. Aufgrund dieser Ausbildung der Kulissenführung wird der kontrollierte und gezielte Anstichvorgang vorgenommen. Dies erfolgt dadurch, dass die Kulissenführung eine in der Gehäusewand unter einer schräg verlaufenden Kulissenbahn umfasst, in der ein an der Wand des Innenteils angeformter Zapfen greift. Wird also das angesetzte Behältnis gegenüber dem Trägerelement gedreht, welches in dem Innenteil angedockt hat, so ergibt sich eine Bewegung des Innenteils in axialer Richtung des eingebundenen Nadelrohrs, wobei der zurück gelegte Weg ausreicht, um insbesondere die Wand des Behältnisses bzw. des Stutzens zu durchstoßen.

Die Ausbildung und Ausgestaltung des Innenteils ist hierbei derart, dass das Innenteil im oberen Bereich eine hülsenartige Aufnahme für den Entnahmebereich des Behältnisses aufweist. Nach einer besonderen Ausführung sind in der hülsenartigen Aufnahme Führungen für den Entnahmebereich des Behältnisses vorgesehen, die einen begrenzten Einschub des Behältnisses in das Trägerelement gewährleisten, um so einen ungewollten Anstich des Behältnisses zu unterbinden.

Hierbei ist der Entnahmebereich des Behältnisses als zylinderförmiger Stutzen ausgebildet, wobei auf dem Stutzen eine Kappe mit einer am Boden vorgesehenen Membran aufsetzbar ist, die in der hülsenartigen Aufnahme im eingesetzten Zustand verrastet.

Es besteht nun die Möglichkeit, dass die Ampulle mit ihrem stutzenartigen Auslass zunächst mit der Kappe versehen wird, die dann in das Trägerelement eingeführt wird, und zwar derart, dass diese an dem Innenteil in dem Entnahmebereich verrastet. Dadurch ergibt sich einerseits ein sicherer Halt des Stutzens und andererseits ein sicherer Halt der Kappe in dem Innenteil.

Das Innenteil als solches weist im unteren Bereich eine weitere Aufnahme für die Hubeinbindung des Nadelrohrs auf. Dabei ist die Hubeinbindung in der Aufnahme in Raststellungen eingebunden. Diese Ausbildung ermöglicht es nun, dass, wenn das Innenteil um den Verschiebeweg durch Kulissenführung in Achsrichtung des Nadelrohrs verschoben wird, im Gegendruck das Nadelrohrende in eine Rastendstellung verbracht wird, so dass in dieser Rastendstellung der Anstich oder Durchstoß der Nadel in das Behältnis erfolgt.

### Ausführungsbeispiele

Ein Ausführungsbeispiel der Erfindung ist in den Zeichnungen rein schematisch dargestellt und wird nachfolgend näher beschrieben. Es zeigt
- Fig. 1:: eine perspektivische Darstellung der Vorrichtung mit angesetzter Ampulle in Seitenansicht,
- Fig. 2:: eine weitere Darstellung gemäß der Figur 1 der Vorrichtung mit verschwenktem Schutzgehäuse,
- Fig. 3:: eine geschnittene Seitenansicht der Vorrichtung im nicht durchstoßenen Zustand der Nadel,
- Fig. 4:: eine weitere geschnittene Darstellung gemäß der Figur 3 im durchstoßenen Zustand der Nadel und
- Fig. 5:: eine weitere Ausführungsform gemäß der Erfindung mit abgeschwenktem Gehäuse einer weiteren Ausführungsform.

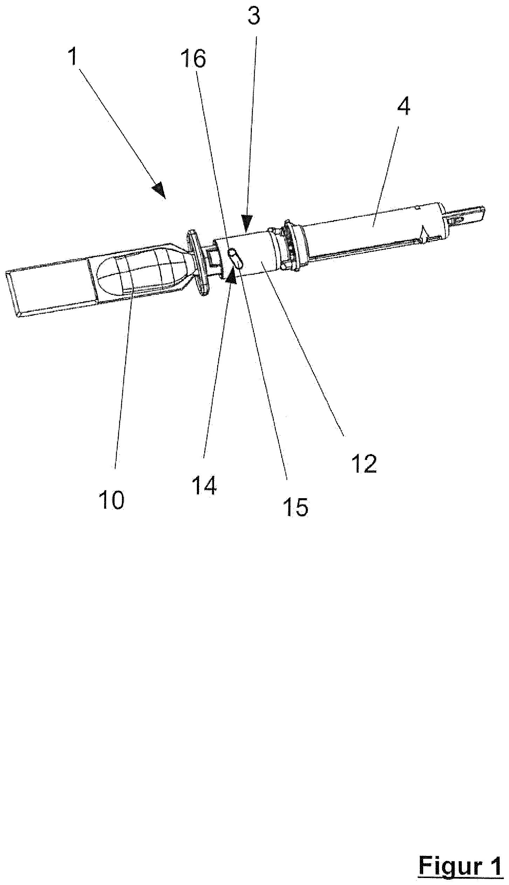

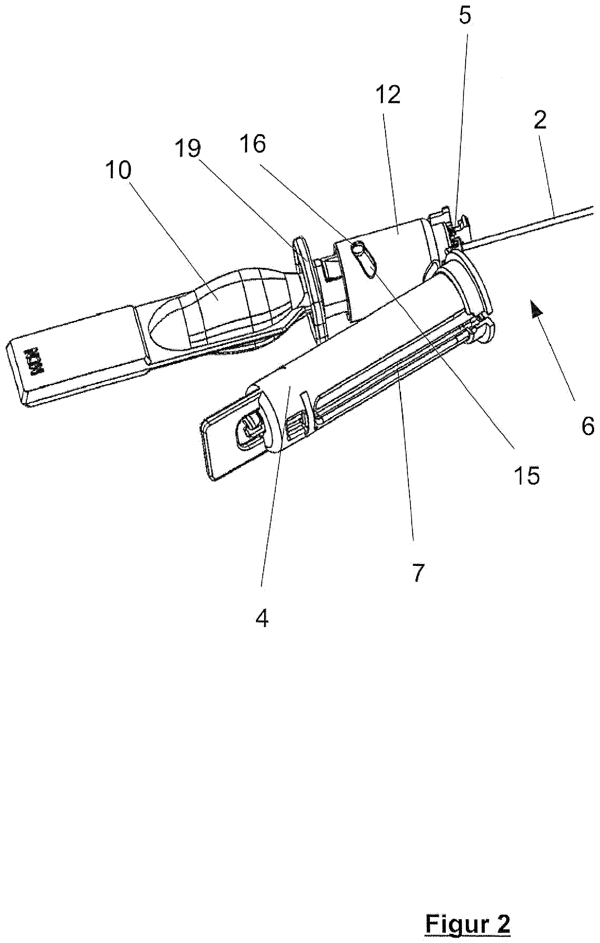

Die **Figuren 1** **und** **2** zeigen jeweils in unterschiedlichen Zuständen eine Vorrichtung **1** für das Nadelrohr **2** einer Spritze, welche ein an einem Trägerelement **3** für das Nadelrohr **2** schwenkbar ausgebildetes Gehäuse **4** umfasst. Wie insbesondere in der **Figur 2** erkennbar ist, kann das Gehäuse **4** um eine Schwenkachse **5** gegenüber einem Trägerelement **3** derart verschwenkt werden, dass das Nadelrohr **2** für die Injektion freiliegt. Das Gehäuse **4** weist hierbei in der Schwenkebene **6** des Gehäuses **4** eine offene Gehäuseseite **7** auf, so dass das Nadelrohr **2** nach Injektionsgebrauch, wie dies in der **Figur 1** dokumentiert ist, zur Aufnahme in das Gehäuse **4** eingeschwenkt werden kann.

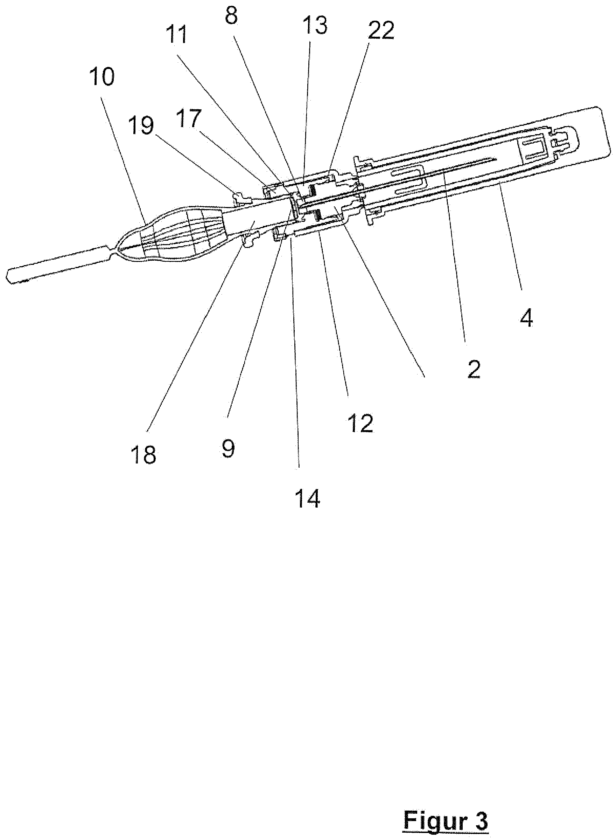

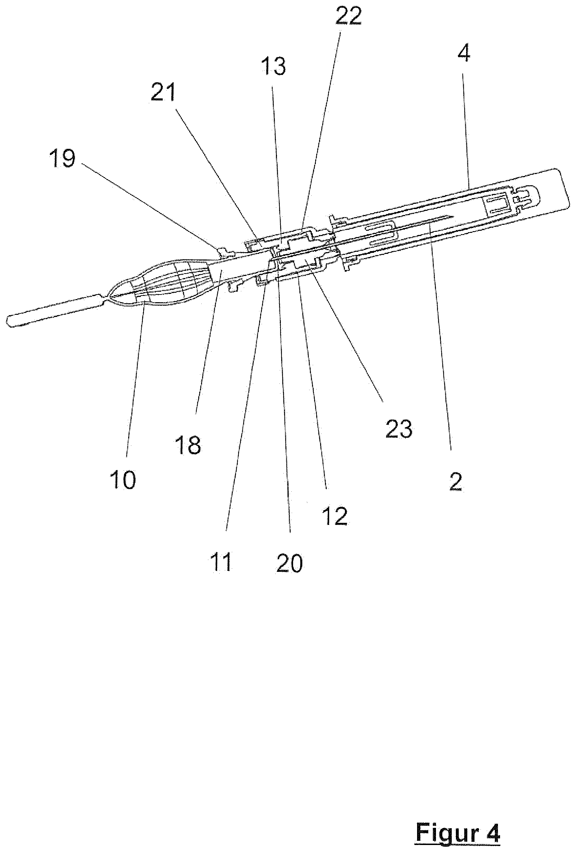

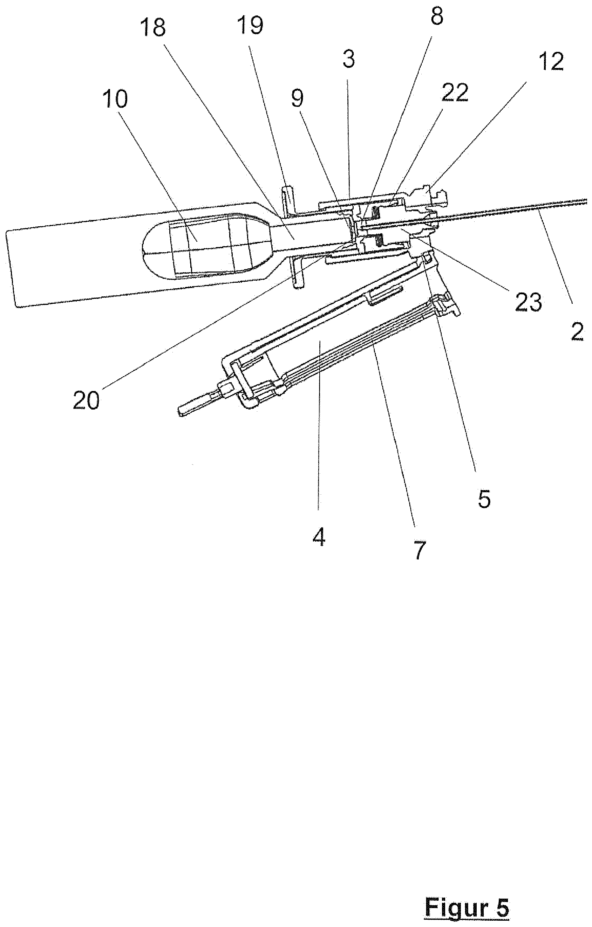

Wie aus den **Figuren 1** **und** **2** erkennbar, ist hierbei die Anordnung des rückwärtigen freien Endes **8** des Nadelrohrs **2** im Trägerelement **3** derart angeordnet, dass ein kontrolliertes Durchstoßen der Wand **9** eines in das Trägerelement **3** eingesetzten Behältnisses **10** ermöglicht wird, wie dies in dem Zustand in der **Figur 4** dokumentiert ist. Dort ist zu sehen, dass die Nadelrohrspitze **11,** die leicht abgeschrägt ist, in das Behältnis **10** eingedrungen ist. Der Vorgang des Durchdringens bzw. des Anstichs wird hierbei dadurch erreicht, dass das rückwärtige freie Ende **8** des Nadelrohrs **2** durch die Schwenk- und Drehbewegung des eingesetzten Behältnisses **10** gegenüber dem Trägerelement **3** erfolgt. Um dies zu ermöglichen, umfasst das Trägerelement **3** ein Gehäuse **12,** in dem ein drehbar gelagertes und darin verschiebbares Innenteil **13** angeordnet ist. Dies ist insbesondere in den **Figuren 3** **und** **4** sowie auch in der **Figur 5** zu erkennen.

Das Innenteil **13** steht hierbei über eine Kulissenführung **14** mit dem Gehäuse **12** in Wirkverbindung, wie dies in den **Figuren 1** **und** **2** näher zu erkennen ist. Die Kulissenführung **14** umfasst hierbei eine in der Gehäusewand unter einer Schrägen verlaufende Kulissenbahn **15,** in der ein an der Wand des Innenteils **13** angeformter Zapfen **16** greift. Es versteht sich nun, wenn man die Seitenansicht der **Figur 1** betrachtet, dass, wenn das Gehäuse **12** des Trägerelements **3** gegenüber dem Behältnis **10** gedreht wird, oder verschwenkt wird, dass in der Kulissenbahn **15** entsprechend der Zapfen **16** geführt wird, wodurch dann eine kontrollierte Betätigung des Innenteils **13** in dem Gehäuse **12** erfolgt, und zwar derart, dass das angesetzte Behältnis **10** einen Verschiebeweg in das Trägerelement **3** zurücklegt.

Wird die Wegstrecke der Kulissenbahn **15** zurückgelegt, so ergibt sich die Situation der **Figur 4**, wo insbesondere das Nadelrohr **2** mit seinem rückwärtigen freien Ende **8** die Wand 9 des Behältnisses **10** durchdringt. In dieser Situation besteht nun die Möglichkeit, dass eine Entleerung der Ampulle vollzogen werden kann, so dass dadurch die vorgehaltene Flüssigkeit aus dem als Behältnis **10** ausgebildeten Ampulle durch das Nadelrohr **2** injiziert werden kann.

In Weiterbildung weist das in dem Gehäuse **12** dreh- und verschiebbar gelagerten Innenteil **13** im oberen Bereich eine hülsenartige Aufnahme **17** für den Entnahmebereich des Behältnisses **10** auf. Der Entnahmebereich des Behältnisses **10** ist hierbei als zylinderförmiger Stutzen **18** ausgebildet. Nach einer besonderen Ausführung sind in der hülsenartigen Aufnahme **17** Führungen für den Entnahmebereich des Behältnisses **10** vorgesehen, die einen begrenzten Einschub des Behältnisses **10** in das Trägerelement **3** gewährleisten, um so einen ungewollten Anstich des Behältnisses **10** zu unterbinden.

Wie insbesondere aus der Zusammenschau der **Figur 3** **und** **4** erkennbar ist, ist auf dem Stutzen **18** eine Kappe **19** mit einer am Boden vorgesehenen Membran **20** aufgesetzt, die in der hülsenartigen Aufnahme **17** eingeführt und im eingesetzten Zustand verrastet ist. Die einzelnen Rasten **21** sind in der **Figur 3** **und** **4** angedeutet, so dass in Folge der angesetzten Kappe **19** eine sichere Einbindung, insbesondere des Stutzens **18** am Trägerelement **3** sichergestellt wird.

Wie insbesondere aus den beiden **Figuren 3** **und** **4** weiter zu erkennen ist, weist das Innenteil **13** im unteren Bereich eine untere Aufnahme **22** für die Hubeinbindung **23** des Nadelrohrs **2** auf. Dabei ist die Hubeinbindung **23** in der Aufnahme **22** in Raststellungen eingebunden, wobei in der zweiten Raststellung insbesondere die Hubeinbindung **23** unmittelbar eine feste Anlage in der Aufnahme **22** des Innenteils **13** einnimmt, so dass dadurch in der Endlage der Hubeinbindung **23** der sichere Einstich durch die Wand **9** erfolgt. Die in den **Figuren 3** **und** **4** dargestellten Raststellungen entsprechen hierbei dem Verschiebeweg des Innenteils **13** in dem Gehäuse **12** des Trägerelementes **3,** wenn die Kulissenführung **14** betätigt wird.

### Bezugszeichenliste

- 1: Vorrichtung
- 2: Nadelrohr
- 3: Trägerelement
- 4: Gehäuse
- 5: Schwenkachse
- 6: Schwenkebene
- 7: Offene Gehäuseseite
- 8: Freies Ende rückwärtig
- 9: Wand
- 10: Behältnis
- 11: Nadelrohrspitze
- 12: Gehäuse Trägerelement
- 13: Innenteil
- 14: Kulissenführung
- 15: Kulissenbahn
- 16: Angeformter Zapfen
- 17: Hülsenartige Aufnahme
- 18: Stutzen Behältnis
- 19: Kappe
- 20: Membran
- 21: Rasten
- 22: Untere Aufnahme
- 23: Hubeinbindung

## Patentansprüche

1. Vorrichtung (1) für das Nadelrohr (2) einer Spritze, umfassend ein an einem Trägerelement (3) für das Nadelrohr (2) schwenkbar ausgebildetes Gehäuse (4), welches in der Schwenkebene (6) des Gehäuses (4) eine offene Gehäuseseite (7) aufweist, so dass das Nadelrohr (2) nach Injektionsgebrauch zur Aufnahme in das Gehäuse (4) einschwenkbar ist,
**dadurch gekennzeichnet, dass**
das Trägerelement (3) ein Gehäuse (12) umfasst, in dem ein drehverschiebbar gelagertes Innenteil (13) angeordnet ist,
wobei das Innenteil (13) über eine Kulissenführung (14) mit dem Gehäuse (4) in Wirkverbindung steht,
wobei die Kulissenführung (14) in der Wand des Gehäuses (12) eine schräg verlaufende Kulissenbahn (15) umfasst, in der ein an der Wand des Innenteils (13) angeformter Zapfen greift, sodass bei einer Drehbewegung eines angesetzten, in dem Innenteil (13) angedockten Behältnisses (10) sich gegenüber dem Trägerelement (3) eine Bewegung des Innenteils (13) in axialer Richtung des eingebundenen Nadelrohrs (2) ergibt,
wobei die Anordnung des rückwärtigen freien Endes (8) des Nadelrohres (2) im Trägerelement (3) infolge des zurück gelegten Weges des Innenteils (13) infolge der Drehbewegung des Behältnisses (10) ein kontrolliertes Durchstoßen der Wand (9) des Behältnisses (10) ermöglicht.

2. Vorrichtung nach Anspruch 1,
**dadurch gekennzeichnet,**
**dass** das Innenteil (13) im oberen Bereich eine hülsenartige Aufnahme (17) für den Entnahmebereich des Behältnisses (10) aufweist.

3. Vorrichtung nach den Anspruch 1 und 2,
**dadurch gekennzeichnet,**
**dass** in der hülsenartigen Aufnahme (17) Führungen für den Entnahmebereich des Behältnisses (10) vorgesehen sind, die einen begrenzten Einschub gewährleisten.

4. Vorrichtung nach den Ansprüchen 1 bis 3,
**dadurch gekennzeichnet,**
**dass** der Entnahmebereich des Behältnisses als zylindrischer Stutzen (18) ausgebildet ist.

5. Vorrichtung nach den Ansprüchen 1 bis 4,
**dadurch gekennzeichnet,**
**dass** auf den Stutzen (18) eine Kappe (19) mit einer am Boden vorgesehenen Membrane (20) aufsetzbar ist, die in der hülsenartigen Aufnahme (17) im eingesetzten Zustand verrastet ist.

6. Vorrichtung nach den Ansprüchen 1 bis 5,
**dadurch gekennzeichnet,**
**dass** das Innenteil (13) im unteren Bereich eine weitere untere Aufnahme (22) für die Hubeinbindung (23) des Nadelrohres (2) aufweist.

7. Vorrichtung nach den Ansprüchen 1 bis 6,
**dadurch gekennzeichnet,**
**dass** die Hubeinbindung (23) für das Nadelrohr (2) in der Aufnahme (22) in Raststellungen eingebunden ist.

## Claims

1. Device (1) for the needle tube (2) of a syringe, comprising a housing (4), which is designed such that it can pivot on a carrier element (3) for the needle tube (2), and which has an open side (7) in the pivot plane (6) of the housing (4), such that after it has been used for injection the needle tube (2) can be pivoted into the housing (4) to be received by the latter,
**characterised in that**,
the carrier element (3) comprises a housing (12), in which an inner part (13), mounted such that it can be moved in a rotational manner, is arranged, wherein the inner part (13) is operatively connected to the housing (4) via a link guide (14), wherein
the link guide (14) in the wall of the housing (12) comprises an obliquely extending link track (15), with which a pin formed on the wall of the inner part (13) engages, such that, in the event of a rotational movement of an attached container (10) docked in the inner part (13), a movement of the inner part (13), relative to the carrier element (3), ensues in the axial direction of the contained needle tube (2), wherein the arrangement of the rear free end (8) of the needle tube (2) in the carrier element (3) enables a controlled piercing of the wall (9) of the container (10) as a result of the travel of the inner part (13) caused by the rotational movement of the container (10).

2. Device according to Claim 1,
**characterised in that**,
in the upper section the inner part (13) has a sleeve-like receptacle (17) for the withdrawal section of the container (10).

3. Device according to the Claims 1 and 2,
**characterised in that**,
guides for the withdrawal section of the container (10) are provided in the sleeve-like receptacle (17), which guides ensure a limited insertion.

4. Device according to the Claims 1 to 3,
**characterised in that**,
the withdrawal section of the container is designed as a cylindrical nozzle (18).

5. Device according to the Claims 1 to 4,
**characterised in that**,
a cap (19), with a membrane (20) provided on the bottom, can be fitted onto the socket (18), which cap, in the inserted state, is latched in the sleeve-like receptacle (17).

6. Device according to the Claims 1 to 5,
**characterised in that**,
in the lower section the inner part (13) has a further lower receptacle (22) for the containment (23) of the stroke of the needle tube (2).

7. Device according to the Claims 1 to 6,
**characterised in that**,
in the receptacle (22) the containment (23) of the stroke for the needle tube (2) is contained in latching positions.

## Revendications

1. Dispositif (1) pour le tube d'aiguille (2) d'une seringue, comprenant un corps (4) conçu sous forme pivotante sur un élément porteur (3) pour le tube d'aiguille (2), lequel dans le plan de pivotement (6) du corps (4) comporte un côté de corps (7) ouvert, de sorte qu'après l'utilisation pour l'injection, le tube d'aiguille (2) puisse se pivoter dans le corps (4) pour y être réceptionné,
**caractérisé en ce que**
l'élément porteur (3) comprend un corps (12) dans lequel est placée une partie intérieure (13) logée en étant déplaçable en rotation,
la partie intérieure (13) étant en liaison active avec le corps (4) par l'intermédiaire d'un guidage coulissant (14),
le guidage coulissant (14) comprenant dans la paroi du corps (12) une trajectoire à coulisse (15) s'écoulant en oblique, sur laquelle s'engage un tenon surmoulé fixé, arrimé dans la partie intérieure (13), de telle sorte que lors d'un déplacement en rotation d'un contenant (10) fixé, arrimé dans la partie intérieure (13), il en résulte par rapport à l'élément porteur (3) un déplacement de la partie intérieure (13) en direction axiale du tube d'aiguille (2) intégré,
du fait du chemin parcouru par la partie intérieure (13) suite au déplacement en rotation du contenant (10), la disposition de l'extrémité (8) arrière libre du tube d'aiguille (2) dans l'élément porteur (3) permettant un une perforation contrôlée de la paroi (9) du contenant (10).

2. Dispositif selon la revendication 1,
**caractérisé**
**en ce que** la partie intérieure (13) comporte dans la zone supérieure un réceptacle (17) en forme de douille pour la zone de prélèvement du contenant (10).

3. Dispositif selon les revendications 1 et 2,
**caractérisé**
**en ce que** dans le réceptacle (17) en forme de douille sont prévus des guidages pour la zone de prélèvement du contenant (10), qui assurent une insertion limitée.

4. Dispositif selon les revendications 1 à 3,
**caractérisé**
**en ce que** la zone de prélèvement du contenant est conçue sous la forme d'une tubulure (18) cylindrique.

5. Dispositif selon les revendications 1 à 4,
**caractérisé**
**en ce que** sur la tubulure (18) peut s'enfoncer un capuchon (19), doté d'une membrane (20) prévue sur le fond inférieur, qui en position introduite est enclenché dans le réceptacle (17) en forme de douille.

6. Dispositif selon les revendications 1 à 5,
**caractérisé**
**en ce que** la partie intérieure (13) comporte dans la zone inférieure un autre réceptacle (22) inférieur, pour l'intégration en course (23) du tube d'aiguille (2) .

7. Dispositif selon les revendications 1 à 6,
**caractérisé**
**en ce que** l'intégration en course (23) pour le tube d'aiguille (2) est intégré dans le réceptacle (22) dans des positions d'enclenchement.
